# EUROPEAN PATENT SPECIFICATION

(11) **EP 0 245 459 B1**
(45) Date of publication and mention of the grant of the patent: **29.09.1993**
(21) Application number: 86907120.9
(22) Date of filing: 05.11.1986
(51) Int. Cl.: C07K 7/10, C07K 15/00, C12Q 1/70, G01N 33/566

(54) **T-CELL LYMPHOTROPHIC VIRUS PROTEIN AND ASSAY**
T-ZELLEN-LYMPHOTROPHISCHES VIRUS-PROTEIN UND TESTVERFAHREN
PROTEINE DE CELLULES INFECTEES PAR LE VIRUS LYMPHOTROPHIQUE DES CELLULES T, ET SON ANALYSE

(30) Priority: 07.11.1985 US 795997
(43) Date of publication of application: 19.11.1987
(73) Proprietor: THE PRESIDENT AND FELLOWS OF HARVARD COLLEGE, Cambridge, MA 02114 (US)
(72) Inventor: ESSEX, Myron, E., North Easton, MA 02356 (US); ALLAN, Jonathan, S., Westwood, MA 02090 (US); LEE, Tun-Hou, Newton, MA 02159 (US)
(74) Representative: Moon, Donald Keith
(86) International application number: US8602381
(87) International publication number: WO8702988

(56) References cited:
- EP-A- 0 187 041
- US-A- 4 520 113
- US-A- 4 554 101
- NATURE, vol. 313, 7th February 1985, pages 450-459; M.A. MUESING et al.: "Nucleic acid structure and expression of the human AIDS/lymphadenopathy retrovirus"
- CELL, vol. 40, January 1985, pages 9-17, MIT, Cambridge, MASSACHUSETTS, US; S. WAIN-HOBSON et al.: "Nucleotide sequence of the AIDS virus, LAV"
- SCIENCE, vol. 230, no. 4727, November 1985, pages 810-813, The American Association for the Advancement of Science, Washington, US; J. S. ALLAN et al.: "A new HTLV-III/LAV encoded antigen detected by antibodies from AIDS patients"
- Proceeding National Academy Science USA vol. 81, pages 6202-6206, issued October 1984
- Proceeding National Academy Science, issued December 1985, vol 82, pages 8359-8363
- Chemical Abstract, vol 104, issued 1986, abstract no. 182928e
- Chemical Abstract, vol 104, issued 1986, abstract no. 1644r
- Chemical Abstract, vol 103, issued 1985, abstract no. 52370k
- Cell, vol. 41, pages 979-986, issued July 1985
- Science, vol. 228, no. 4703, issued 31 May 1985, pages 1091-1094
- Science, vol. 228, issued 31 May 1985, pages 1094-1096

## Description

This invention relates to novel purified forms of a protein found in cells infected with human T-cell lymphotrophic virus III (HTLV-III) and related viruses; the invention also relates to polypeptides, kits, and assays for detecting in a biological specimen the presence of that protein's antigenic determinants or of antibodies to one or more such antigenic determinants. For convenience, the term "HTLV-III related viruses" is used in this application to include viruses that are closely related to HTLV-III by serological biochemical and molecular criteria, including lymphadenopathy associated virus (LAV) (see Barre-Sinoussi (1983) Science 220:859), ARV (see Sanchez-Pescador (1985) Science 227:484) and AAV, and other forms, subtypes and variants, including simian viruses. The terms "human T-cell lymphotrophic virus" and "human T-cell leukemia virus" are used interchangeably, although the former is preferable.

Human T-cell lymphotrophic virus III (HTLV-III) is believed to play a Key role in the pathogenesis of acquired immunodeficiency syndrome (AIDS). It has been shown that human patients whose bodies contain antibodies to HTLV-III-infected cells are apparently latently or actively infected with the virus. There are various tests to determine the presence of antibodies to HTLV-III proteins in a biological specimen. For example, an ELISA test is widely used for blood bank screening. For such assays, the goal is to screen out all individuals who have been exposed to HTLV-III, whether or not such individuals have developed or will develop AIDS.

Other HTLV-III-encoded polypeptides that are antigenic when expressed in infected individuals include:
1) a 55 kd gag polyprotein (p55) which yields a 24 kd protein (p24) as the major virus core protein and a 17 kd phosphoprotein (pp17) (Schupbach et al. (1984) Science 224:503-505); and
(2 a gp160 env polyprotein which gives rise to gp120 at the amino terminus (see Essex and Lee, USSN 670,361, filed November 9, 1984, which is hereby incorporated by reference).

The DNA sequence of the HIV genome and theoretical open reading frames of HIV with resulting putative amino acid sequences have been described by Muesin et al in Nature Vol 313 p450 1985 and EP-A-0187041 and also by Wain-Hobson et al in Cell vol 40 p9 1985.

The disease course for patients who exhibit HTLV-III antibodies varies; some such patients are healthy and currently uninfected, other harbour the virus with few symptoms, still others have chronic symptoms that have been designated "AIDS related complex" (ARC), and, finally, some such individuals are AIDS patients, exhibiting immunodeficiencies that are ultimately fatal. It also appears that some ARC or non-symptomatic patients harbouring the virus may develop AIDS after prolonged periods.

### Summary of the Invention

Cells infected with HTLV-III and related viruses yield a protein coded by the open reading frame (ORF) positioned 3' to the env gene. The protein coded by HTLV-III has an apparent molecular wight of about 27,000 daltons, meaning that, when myristylated, the protein migrates in conventional sodium dodecyl sulfate (SDS) electrophoresis gels as described below in a manner that is characteristic of a molecular weight of about 27,000 daltons, even though the actual molecular weight may differ somewhat from 27 kd; when computed from the viral coding sequence, the HTLV-III protein's molecular weight may be lower, e.g., about 22,000 daltons. For convenience, we refer to the protein coded by the open reading frame 3' to the env gene of HTLV-III and HTLV-III related viruses as "p27". The presence of p27, p27 antigenic determinants, or antibodies to p27 antigenic determinants in patients that are positive for HTLV-III or related viruses is useful information (by itself or as part of a panel of tests) for predicting the course of the infection in that patient.

As noted above the presence of p27 antibodies provides useful information concerning the course of disease in individuals that are positive for HTLV-III and related viruses. Accordingly the present invention provides a method of assaying a biological specimen for the presence or antibody to cells infected with HTLV-III or a related virus, said method comprising incubating said specimen with a polypeptide having an antigenic determinant or determinants immunologically cross-reactive with determinants of p27, a protein encoded by the open reading frame 3' to the env gene of HTLV-III or a related virus, and determining whether or not an immunocomplex is formed between said antibody and said polypeptide.

In preferred embodiments of the invention, the assay for p27 antibodies is performed as part of a panel of tests for HTLV-III and related virus antibodies; thus, one or more specimens from the same patient is assayed for antibodies to other HTLV-III and related virus proteins using techniques described in the above-cited references: p55, p24, p17, gp160, gp120, and gp41. The resulting information from positive patients provides an improved basis for estimating the course of the disease. For example, individuals exhibiting either p27 antibodies or p55/p24 antibodies are less likely to have contracted AIDS or ARC than individuals who are negative for both p55 and p24 antibodies; and those who are positive for both antibodies are even less likely to have contracted AIDS or ARC.

In a second aspect the invention provides an assay for the presence of a p27 antigenic determinant or for determinants cross-reactive with the determinants of p27 which comprises: incubating said specimen with antibodies against an antigenic determinant or determinants of said p27, and determining whether or not an immunocomplex is formed between said antibodies and said antigenic determinant or determinants. The determinants to be assayed may occur on p27 itself or on other polypeptides. The determinants may be in free circulation in body fluids or in lymphocytes. The assay can be carried out by known immunoassay methods, using antibodies, monoclonal or polyvalent, having immune reactivity with the antigenic determinants found on p27. For example competitive immunoassays or immunometric (sandwich) assays can be used.

The substantially pure polypeptides that are useful in the present invention contain at least one antigenic determinant that is immunologically cross-reactive with the determinants of a p27 protein obtained from infected cells. By "polypeptides containing immunologically cross-reactive determinants" is meant polypeptides having in common antigenic determinants with which a given antibody will react.

Polypeptides which may be used in the present invention include the p27 protein itself either myristylated or unmyristylated. If myrystylated at the NH₂ terminal, the polypeptide is blocked against Edman degradation. The polypeptides can also include myristylated or unmyristylated fragments of p27. Other useful polypeptides which have the necessary immunogenic determinants include peptides coded by HTLV-III and related viruses as well as synthetic polypeptides, e.g. produced by genetically engineered cells. Preferably the polypeptide is labelled for use in the assay as described below.

Polypeptides which may be used in the present invention also include antibodies, or fragments thereof, which are anti-idiotypic towards the active determinant or determinants on the p27 protein of the invention. It has been shown that anti-idiotypic reagents are useful as diagnostic tools for the detection of antigens carrying sites which are immunologically cross-reactive with those on the antibodies (Potocnjak et al., Science 215: 1637-1639(1982)). Thus, an assay for p27 antibodies could be carried out with the aid of an anti-idiotypic antibody or immunologically active fragment thereof which carries an antigenic site or sites thereon which are immunologically similar to the antigenic site or sites on p27. Such antiidiotypic antibodies can be raised against primary antibodies having specificity against the antigenic sites on the p27 protein of the invention (i.e. the antiidiotypic antibodies are anti-antibodies). Preferably monoclonal anti-idiotypic antibodies are used.

Other features and advantages of the invention will be apparent from the following description of the preferred embodiment and from the claims.

### Description of the Preferred Embodiment

### Drawings

Fig. 1 is a diagrammatic representation of HTLV-III DNA indicating coding regions of various proteins.

Fig. 2 is a graph depicting radioactivity detected in peptide fragments of p27 and depicting the amino terminal amino acid sequence of two such peptide fragments.

Fig. 3 is a photograph of SDS-polyacrylamide gels of radioimmunoprecipitates of HTLV-III infected cell proteins.

### The Protein

The protein of the present invention, when myristylated, has a molecular weight of approximately 27,000 daltons as determined by sodium dodecyl sulfate (SDS) gel electrophoresis and is soluble in SDS buffer consisting of 0.15 M sodium chloride, 0.05 M Tris hydrochloride pH 7.2, 1% Triton X-100, 1% sodium deoxycholate, 0.1% sodium dodecylsulfate, and 1 mM phenylmethylsulfonyl fluoride. Triton X-100 is a nonionic detergent (octylphenoxy polyethoxy (9-10) ethanol). The p27 protein undergoes post-translational myristylation, and is not glycosylated. The unmyristylated moiety of the protein contains substantially the same antigenic determinant or determinants as does the myristylated protein. An NH₂-terminal myristylate group blocks Edman degradation.

Further characterization of the p27 protein, and indication that it is distinct from other, previously described HTLV-III specific proteins listed above, can be obtained by mapping the protein to HTLV-III DNA. p27 protein is obtained as described below from HTLV-III infected cells labeled with [³H] leucine and [³H] valine. The protein is fragmented with cyanogen bromide, and the fragments are separated by Sephacyl S-200 chromatography. Fractions are analyzed by Edman degradation and pooled.

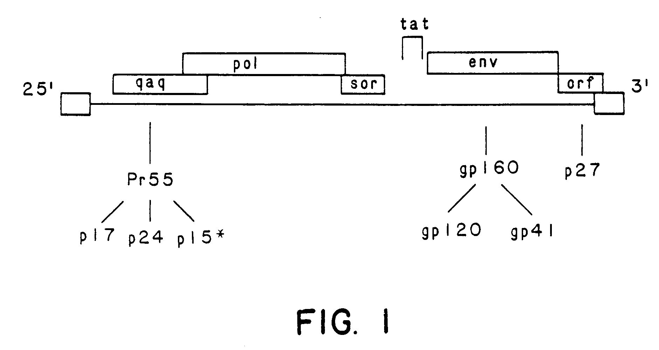

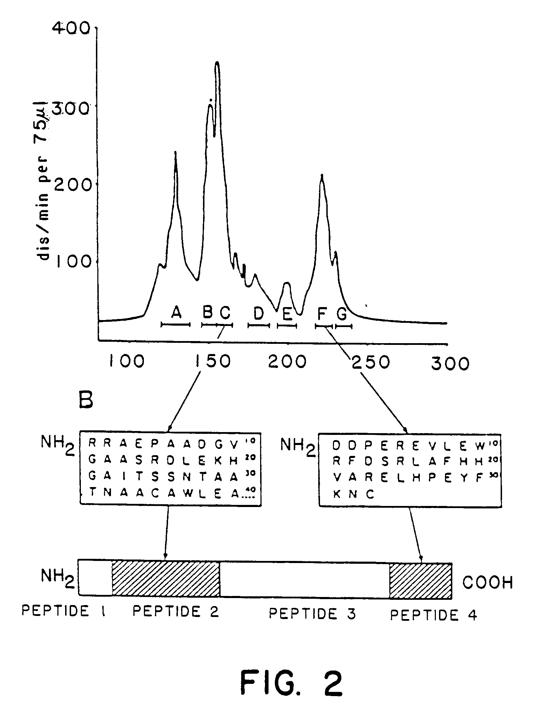

Fig. 2 shows a diagrammatic representation of four peptides that are predicted to be generated by cyanogen bromide cleavage at met residues at positions 1, 20, 60, and 170 of the 204 amino acid sequence deduced from the 3' ORF of HTLV-III DNA. To match p27 to that HTLV-III DNA coding region, H9/HTLV-III_{E-84-4} cells (described below) are metabolically labeled with 10mCi of [³H] leucine or [³H] valine for four hours and cell lysates prepared. Radiolabeled lysates are precipitated with standard HTLV-III reference serum (same as Fig. 1, lane 2) and electrophoresed on 10% SDS-polyacrylamide gels. The p27 is excised from the gel, electroeluted and lyophilized prior to cyanogen bromide cleavage. Cyanogen bromide hydrolysis of p27 is accomplished by incubating the protein for nine hours with two percent CNBr in seventy percent formic acid. The reaction is terminated with the addition of nine volumes of water and the mixture is lyophilized. Sephacryl S-200 separation of CNBr peptides and automated sequence analysis are performed by the general technique of Lee et al., Science (1984) 226:57 et seq. and Coligan et al. Methods Enzymol. (1983) 91:413. Fractions are collected, assayed for radioactivity, pooled, and subjected to Edman degradation. The sequences derived from pools C and F are compared to the amino acid sequences of CNBr cleavage products of the gene product coded by the 3' open reading frame (ORF) of HTLV-III derived from the DNA sequence data given by Meusing et al. (1985) Nature (London) 313:459 or Sanchez-Pescador (1985) Science 227:484.

The amino terminal sequence data derived from Edman degradation of peptides actually derived from pools C and F matches the predicted sequences shown in Fig. 2. Accordingly, the coding region for p27 is the open reading-frame (ORF) at the 3' terminal region of HTLV-III. The labeled amino acids are indicated by asterisks. It is likely that the methionine initiator sequence for the p27 coding region is the AUG codon in the 3' OFF which begins 5 nucleotides from the 3' end of the env gene and is followed by a glycine codon.

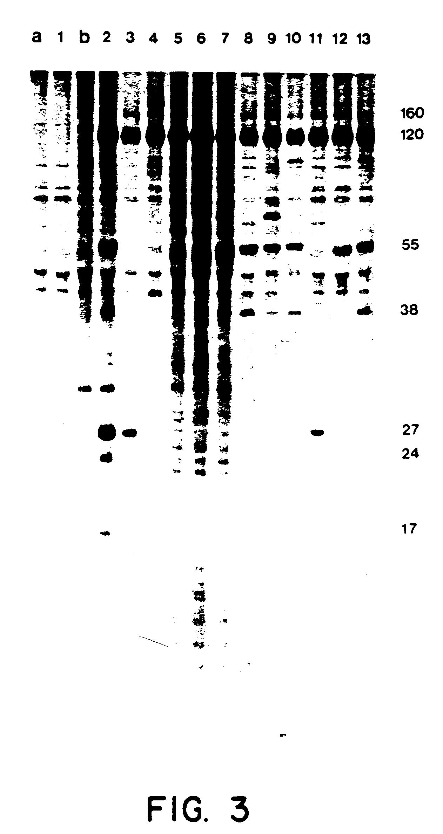

Fig. 3 is a photograph of an SDS-Page radioimmunoprecipitation of HTLV-III proteins showing antibody reactivities specific to p27. Cell lysates are prepared from [³⁵S] cysteine labeled H9/HTLV-III_{E-84-4} cells or H9 uninfected cells. Radioimmunoprecipitation and SDS-PAGE are carried out by the method generally described in Kitchen et al. (1984) Nature 312:367; Barin et al. (1985) Science 228:1094; and Allan et al. (1985) Science 228:1091. Lysates are immunoprecipitated with the following sera: normal human serum pre-tested on uninfected H9 cells (lane a) and H9/HTLV-III_{E-84-4} cells (lane 1); positive reference serum from an ARC patient pre-tested on uninfected (lane b) and infected cells (lane 2); serum from seven ARC patients (lanes 3, 4, 5, 8, 9, 10, and 13); serum from three AIDS patients (lanes 6, 11, and 12); and serum from one healthy homosexual male (lane 7). The serum of lanes 3-12 is tested on infected H9/HTLV-III_{E-84-4} cells. Immunoprecipitates are electrophoresed on 20 centimeter 10% SDS-polyacrylamide gels.

### Obtaining the Protein

The protein can be obtained from HTLV-infected cells. A variety of cell lines have been prepared, which are permanently and persistently infected with HTLV; among them can be mentioned HTLV-III-infected H9 cells, Lymphadenopathy associated virus (LAV)-infected NC37 cells, and Molt 3 and HUT78 cells infected with fresh AIDS virus isolates. One particular such HTLV-III infected cell line is H9G/HTLV-III_{E-84-4} (ATCC 8983), an isolate produced by repeated passage of H9/HTLV-III (ATCC 8543). It may be that the exact sizes of the novel protein is slightly different in different lines; however, the common immunologically cross-reactive portion of the protein is the same regardless of cell line, since it is a protein induced by HTLV. Thus, any cell which harbors the virus may be an appropriate source for the novel proteins.

In order to obtain the protein from any infected cells carrying the virus, the cells are metabolically labeled (e.g. with ³⁵S-cysteine) and immunoprecipitated with antisera obtained from HTLV-III-infected subjects. The novel proteins can then be detected and isolated in the cell lysate by SDS gel electrophoresis.

A specific protocol of p27 preparation is as follows. Infected HTLV-III cells are harvested at their log phase of growth. After one wash with RPMI-1640 medium, each sample of the cells is resuspended in a labeling medium consisting of RPMI-1640 medium, 10% fetal bovine serum, and 100 µ Ci/ml of ³⁵S-methionine. At the end of about 4 to 8 hours pulsing, the radioactive labeled cells are washed three times with cold PBS. The cell pellet is then lysed with 0.2 ml/1 x 10⁶ cells of cold lysis buffer (RIPA) (0.15 M sodium chloride, 0.05 M Tris-hydrochloride pH 7.2, 1% Triton X-100 wetting agent, 1% sodium deoxycholate, 0.1% sodium dodecyl sulfate, and 1 mM phenylmethylsulfonyl fluoride) After 10 minutes of intermittent vortexing, the mix is centrifuged for 1 hour at 100,000 xg at 4°C and absorbed on Sepharose Cl-4B-protein-A beads. The lysate is then subjected to immunoprecipitation with positive anti-sera, and the immunoprecipitate is electrophoresed.

An alternative method for preparing the protein, the 3' ORF HTLV-III region can be isolated and cloned for expression in a bacterial expression system such generally using the technique described in Chang et al. (1985) Nature 315:151.

### Assays

The purified and isolated proteins or any polypeptide antigen immunologically cross-reactive therewith can be employed as a standard antigen in any conventional assay procedure for detection in biological specimens of the presence of antibodies specific thereto.

The proteins or polypeptides immunologically cross-reactive therewith can be labeled by conventional procedures with ¹²⁵I or ³⁵S or ³H for use in radioimmunoassay, with fluorescein for flourescent immunoassay, with enzyme for enzyme immunoassay or with biotin, for biotin-avidin linked assays. It can be employed, labeled or unlabeled as desired, in competitive immunoassays, as well as in double antibody assays using two antibodies, either of the idiotype:anti-idiotype variety or more particularly of the second antibody type using an anti-Fc antibody, or other assays.

To form the protein antibody complex, aliquots of protein-A-coated beads are bound to: (a) positive reference blood serum from an individual known to harbor antibodies against HTLV-III viral proteins; (b) negative control serum from individuals free from infection; and (c) serum from unknown individuals to be tested. Each aliquot of coated beads is then reacted with an aliquot of each precleared lysate obtained from cells according to the methods described above at 4°C for 4-8 hours to permit complex formation or immunoprecipitation to occur between the bonded lysate protein and any antibody present in the sera. At the end of the reaction the beads are washed 5 times with the buffer (RIPA) to remove uncomplexed lysate protein.

The beads are then immersed in a sample buffer (0.1 M Cleland's reagent, 2% sodium dodecylsulfate, 0.08 M Tris-hydrochloride pH 6.8, 10% glycerol, and 0.2% Bromphenol Blue) and subjected to boiling at 100°C for 2 minutes to elute proteins from the beads and to dissociate complexes.

Alternatively, the novel proteins or polypeptides immunologically cross-reactive therewith could be immobilized on an insoluble phase, such as an insoluble resin, and detection of the anti-protein antibodies can be carried out by measuring their binding to the insoluble phase. Insoluble phases also include latex particles, which when coated with the novel protein or its immunologically cross-reactive polypeptides and subjected to anti-protein antibody, will agglutinate. Yet other insoluble phases include test tubes, vials, titration wells, and the like, to which the novel protein or its immunologically cross-reative polypeptide can be bound, and antibody thereto detected by double antibody techniques or Protein-A dependent techniques.

The assay for p27 antibody may utilize the p27 protein in crude form and is not limited to using this protein in substantially pure form. For example, the protein may be substantially purified, or cruder mixtures can be used.

Preferably, the assay for p27 or p27 antibodies is performed on individuals who are seropositive, that is, who demonstrate the presence of antioodies to one of the relatively strongly antigenic glycoproteins (gp120, gp160, and gp41). The p27 antibody assay provides the following information:

| Disease Course for HTLV-III (gp120) Seropositive Individuals | | | | |
|---|---|---|---|---|
| | Symptom-free | ARC | AIDS | Total |
| anti-p27⁺ | 48.1% | 42.9% | 29.0% | 37.0 |

Also, preferably, knowledge that an HTLV-III positive patient has anti-p27 antibodies is combined with information gained from other tests to increase the reliability of an assessment of the course of the virus in that patient. For example, the presence of antibodies to other weakly antigenic HTLV-III or related virus proteins (e.g., p55 or p24) is further information to be used with the presence of anti-p27 antibodies. Such tests can be used as a panel to predict the disease course.

Preferably, the tests for the various HTLV-III antibodies are performed by the methods indicated in the following table:

| Antigen | Genomic Origin in Virus | Richest Source* | Optimal Procedures for Antibody Testing* |
|---|---|---|---|
| P⁵⁵ | gag (entire) | IC* | WBIC, IPIC, CIF* |
| p²⁴ | gag (middle) | CV | E, WBCV, WBIC, ICV, IPIC, CIF |
| p²⁷ | 3' orf (entire) | IC | IPIC |

| | | | |
|---|---|---|---|
| *Abbreviations: CIF, cytoplasmic immunofluorescence; CV, concentrated virus; E, ELISA; IC, infected cell; ICV, immunoprecipitation with concentrated virus; IPIC, immunoprecipitation with infected cell homogenate; WBIC, Western Blotting with infected cell homogenate; WBCV, Western blotting with concrated virus. | | | |

The elements necessary for carrying out the diagnostic methodology described hereinbefore may be present in a kit. Such kit comprises a carrier being compartmentalized to receive therein one or more containers, each of said containers comprising one or more elements necessary to carry out the tests.

For example, the first container may contain one or both of the purified protein p27 or its immunologically cross-reactive polypeptides in detectably labeled or in insolubilized form.

A second container may comprise anti IgG antibody, polyclonal or monoclonal, useful in double antibody binding assay, or elements needed for detection of the label on the protein or its immunologically cross-reactive polypeptides (e.g. chromogenic substrates).

Additional containers may comprise varying amounts of the protein or its immunologically cross-reactive polypeptides which can be used to prepare a standard curve into which experimental results can be interpolated. The materials may be present in the kit by themselves, in solution, freeze-dried, or in admixture with other inert materials, such as inert proteins, and the like.

The biological specimens tested may include blood, serum, lymphocytes, urine, tissues, saliva, feces, and the like. Other protein antibodies may be added to the test panel, for example, antibodies to proteins coded by viruses that are closely related to HTLV-III.

## Claims

1. A method of assaying a biological specimen for the presence of antibody to cells infected with HTLV-III or a related virus, said method comprising incubating said specimen with a polypeptide having an antigenic determinant or determinants immunologically cross-reactive with determinants of p27, a protein encoded by the open reading frame 3' to the env gene of HTLV-III or a related virus, and determining whether or not an immunocomplex is formed between said antibody and said polypeptide.

2. A method according to claim 1 wherein the polypeptide is labelled.

3. A method according to claim 2 further comprising assaying said biological specimen for the presence of a second antibody to cells infected with HTLV-III or a related virus by incubating said specimen with a second polypeptide and determining whether or not an immunocomplex is formed between said second antibody and said second polypeptide, said second polypeptide having an antigenic determinant immunologically cross-reactive with determinants of a second protein obtained from the cells infected with HTLV-III or a related virus, said second polypeptide lacking antigenic determinants immunologically cross-reactive with said p27 or said first polypeptide.

4. A method according to claim 3 wherein said second polypeptide is a gag polypeptide or an env polypeptide.

5. A method according to claim 4 wherein said second polypeptide is selected from p55, p24, pp17, gp160, gp120 and gp41.

6. A method according to claim 5 wherein said second protein is p55.

7. A method according to any preceding claim wherein the polypeptide is p27 or a fragment thereof.

8. A method of detecting the presence in a biological specimen of an antigenic determinant or determinants immunologically cross-reactive with protein p27, a protein encoded by the open reading frame 3' to the env gene of HTLV-III or a related virus which comprises:
incubating said specimen with antibodies against an antigenic determinant or determinants of said p27, and
determining whether or not an immunocomplex is formed between said antibodies and said antigenic determinant or determinants.

9. A method according to claim 8 wherein said specimen comprises human lymphocytes.

10. A kit for assaying a sample for the presence of antibody to cells infected with HTLV-III or a related virus, said kit comprising a first container containing a polypeptide having at least one antigenic determinant or determinants immunologically cross-reactive with protein p27, a protein encoded by the open reading frame 3' to the env gene of HTLV-III or a related virus and
a second container containing means for detecting the formation of an immunocomplex between said antibody and said polypeptide.

## Patentansprüche

1. Verfahren zum Testen biologischer Proben auf das Vorhandensein von Antikörpern gegen Zellen, die mit HTLV-III oder einem verwandten Virus infiziert sind, bei dem die Probe mit einem Polypeptid inkubiert wird, das eine antigene Determinante oder Determinanten aufweist, die mit den Determinanten von p27 immunologisch crossreagieren , einem Protein, das durch den offenen 3'-Leseraster zum env-Gen von HTLV-III oder einem verwandten Virus codiert wird, und bei dem festgestellt wird, ob zwischen dem Antikörper und dem Polypeptid ein Immunokomplex gebildet wird oder nicht.

2. Verfahren nach Anspruch 1, bei dem das Polypeptid markiert ist.

3. Verfahren nach Anspruch 2, das ferner das Testen der biologischen Probe auf das Vorhandensein eines zweiten Antikörpers gegen Zellen umfaßt, die mit HTLV-III oder einen verwandten Virus infiziert ist , indem die Probe mit einem zweiten Polypeptid inkubiert und festgestellt wird, ob zwischen dem zweiten Antikörper und dem zweiten Polypeptid ein Immunokomplex gebildet wird oder nicht, wobei das zweite Polypeptid eine antigene Determinante aufweist, die mit Determinanten eines zweiten Proteins imnunologisch crossreagieren, das aus mit HTLV-III oder einem verwandten Virus infizierten Zellen gewonnen ist, und wobei dem zweiten Polypeptid Antigendeterminanten fehlen, die mit dem p27 oder dem ersten Polypeptid immunologisch crossreagieren.

4. Verfahren nach Anspruch 3, bei dem das zweite Polypeptid ein gag-Polypeptid oder ein env-Polypeptid ist.

5. Verfahren nach Anspruch 4, bei dem das zweite Polypeptid aus p55, p24, pp17, gp160, gp120 und gp41 ausgewählt ist.

6. Verfahren nach Anspruch 5, bei dem das zweite Protein p55 ist.

7. Verfahren nach einem der vorhergehenden Ansprüche, bei dem das Polypeptid p27 oder ein Fragment hiervon ist.

8. Verfahren, um festzustellen, ob in einer biologischen Probe eine antigene Determinante oder Determinanten vorhanden sind, die immunologisch mit dem Protein p27 crossreagieren, einem Protein, das durch den offenen 3'-Leseraster zum env-Gen von HTLV-III oder einem verwandten Virus codiert ist, dadurch gekennzeichnet, daß
die Probe mit Antikörpern gegen eine antigene Determinante oder Determinanten von p27 inkubiert werden, und daß
festgestellt wird, ob zwischen den Antikörpern und der Antigen-Determinante oder den Determinanten ein Immunokomplex gebildet wird.

9. Verfahren nach Anspruch 8, bei dem die Proben menschliche Lymphozyten umfassen.

10. Zusammenstellung zum Testen einer Probe auf das Vorhandensein von Antikörpern gegen Zellen, die mit einem HTLV-III oder einem verwandten Virus infiziert sind, wobei die Zusammenstellung einen ersten Behälter aufweist, der ein Polypeptid enthält, das wenigstens eine antigene Determinante oder Determinanten hat, die mit dem Protein p27, einem Protein, das durch den offenen 3'-Leseraster zu dem env-Gen von HTLV-III oder einem verwandten Virus codiert ist, immunologisch crossreagiert, und der
einen zweiten Behälter umfaßt, der Mittel enthält, um die Bildung eines Immunokomplexes zwischen dem Antikörper und dem Polypeptid festzustellen.

## Revendications

1. Procédé de dépistage de la présence d'un anticorps dirigé contre des cellules infectées par un virus HTLV-III ou par un virus apparenté dans un échantillon biologique, ledit procédé comprenant une incubation dudit échantillon avec un polypeptide possédant un ou plusieurs déterminants antigéniques présentant une réactivité immunologique croisée avec des déterminants de la protéine p27 qui est une protéine codée par la phase de lecture ouverte en 3' du gène env de HTLV-III ou d'un virus apparenté, et une étape destinée à déterminer s'il se forme ou non un immunocomplexe entre ledit anticorps et ledit polypeptide.

2. Procédé selon la revendication 1 dans lequel le polypeptide est marqué.

3. Procédé selon la revendication 2 comprenant en outre le dépistage dans ledit échantillon biologique de la présence d'un second anticorps dirigé contre des cellules infectées par HTLV-III ou par un virus apparenté en incubant ledit spécimen avec un second polypeptide en déterminant s'il se forme ou non un immunocomplexe entre ledit second anticorps et ledit second polypeptide ledit second polypeptide possédant un déterminant antigénique présentant une réactivité immunologique croisée avec des déterminants d'une seconde protéine provenant de cellules infectées par HTLV-III ou par un virus apparenté, ledit second polypeptide ne possédant pas de déterminants antigéniques présentant une réactivité immunologique croisée avec ladite protéine p27 ou avec ledit premier polypeptide.

4. Procédé selon la revendication 3 dans lequel ledit second polypeptide est un polypeptide gag ou un polypeptide env.

5. Procédé selon la revendication 4 dans lequel ledit second polypeptide est choisi parmi les polypeptides p55, p24, pp17, gp160, gp120 et gp41.

6. Procédé selon la revendication 5 dans lequel ledit second proteine est p55.

7. Procédé selon l'une des revendications précédentes dans lequel le polypeptide est p27 ou un fragment de p27.

8. Procédé de détection dans un échantillon biologique de la présence d'un ou de plusieurs déterminants antigéniques présentant une réactivité immunologique croisée avec la protéine p27 qui est une protéine codée par la phase de lecture ouverte en 3' du gène env de HTLV-III ou d'un virus apparenté qui comprend
l'incubation dudit spécimen avec des anticorps dirigés contre un ou plusieurs déterminants antigéniques de ladite protéine p27 et
une étape visant à déterminer s'il se forme ou non un immunocomplexe entre lesdits anticorps et le ou lesdits déterminants antigéniques.

9. Procédé selon la revendication 8 dans lequel ledit spécimen comprend des lymphocytes humains.

10. Nécessaire pour le dépistage dans un échantillon de la présence d'un anticorps dirigé contre les cellules infectées par HTLV-III ou par un virus apparenté, ledit nécessaire comprenant un premier récipient contenant un polypeptide possédant au moins un ou plusieurs déterminants antigéniques présentant une réactivité immunologique croisée avec la protéine p27 qui est une protéine codée par la phase de lecture ouverte en 3' du gène env de HTLV-III ou d'un virus apparenté et
un second récipient contenant des produits permettant de détecter la formation d'un immunocomplexe entre ledit anticorps et ledit polypeptide.
